(19) Europäisches Patentamt
European Patent Office
Office européen des brevets

(11) Publication number: **0 168 025**
**B1**

(12) **EUROPEAN PATENT SPECIFICATION**

(45) Date of publication of patent specification: **27.09.89**

(51) Int. Cl.⁴: **C 07 D 277/38,** C 07 D 277/40, C 07 D 277/42

(21) Application number: **85108491.3**

(22) Date of filing: **09.07.85**

# (54) Process for producing aminothiazolylpropendicarboxylic acid monoesters.

(30) Priority: **12.07.84 JP 145794/84**

(43) Date of publication of application:
**15.01.86 Bulletin 86/03**

(45) Publication of the grant of the patent:
**27.09.89 Bulletin 89/39**

(84) Designated Contracting States:
**CH DE FR IT LI NL**

(56) References cited:
**EP-A-0 081 674**
**US-A-4 426 528**
**CHEMICAL ABSTRACTS, vol. 100, no. 15, April 9, 1984, Columbus, Ohio, USA. SAGAMI CHEMICAL RESEARCH CENTER: "Isomerisation of (halovinyl) thiazole derivatives", page 593, column 1, abstract n. 121 053m & Jpn. Kokai Tokkyo Koho JP 58 172 385 83 172 383r 11 Oct. 1983**

(73) Proprietor: **SHIONOGI & CO., LTD.**
**1-8, Doshomachi 3-chome Chuo-ku**
**Osaka 541 (JP)**

(72) Inventor: **Matsumura, Hiromu**
**15-10-839, Asahigaoka-cho**
**Ashiya-shi Hyogo (JP)**
Inventor: **Yano, Toshisada**
**5-19-18-Kitaochiai Suma-ku**
**Kobe-shi Hyogo (JP)**

(74) Representative: **Bruin, Cornelis Willem et al**
**Octrooibureau Arnold & Siedsma Isartorplatz 5**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process of producing aminothiazolylpropenedicarboxylic acid monoesters which are useful as starting materials for providing the amido side chain of antibacterial penicillins or cephalosporins and as agents for modifying the amino group of arylgylcylpenicillins or cephalosporins.

The desired compounds as well as a few production methods thereof have been disclosed already in EP—A—0136721. The present invention provides another route of synthesis in which the final step (a mono-esterification) is especially important and which can thus be described either as a five-step or as a one-step method.

In one aspect, the invention relates to a process of producing aminothiazolylpropenedicarboxylic acid mono-esters, which is characterized by the steps of:

1) reacting a haloacetylmalonic acid diester (I) with thiourea to give a 2-(2-amino-4-thiazolyl)malonic acid diester (II) according to the reaction scheme:

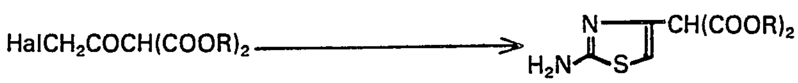

(I) (II)

2) reacting the compound (II) with a amino-protecting reagent to give a 2-(2-protected amino-4-thiazolyl)malonic acid diester (III) according to the reaction scheme:

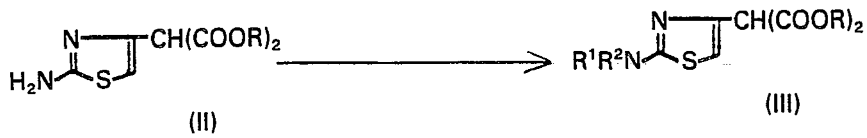

(II) (III)

3) reacting the compound (III) with a haloalkenoic acid ester to give a 1-(2-protected amino-4-thiazolyl)-2-propene-(1,1,3-tri or 1,1,3,3-tetra)carboxylic acid tri- or tetraester (IV), according to the reaction scheme:

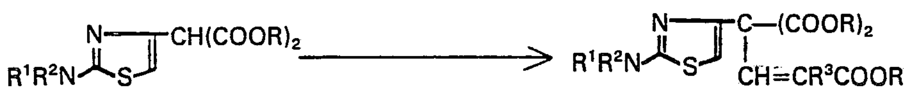

(III) (IV)

4) subjecting the compound (IV) to hydrolysis and dicarboxylation to give a 1-(2-protected amino-4-thiazolyl)propene-1,3-dicarboxylic acid (V), according to the reaction scheme

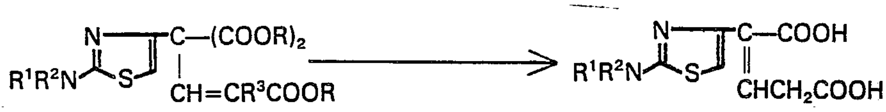

(IV) (V)

5) reacting the compound (V) with an alcohol selected from a (C1—C5)alkanol; (C2—C8)alkenol; (C1—C5)alkanol substituted by halogen or (C1—C5)alkoxy or sulfonyl; benzylalcohol; or benzylalcohol substituted by (C1—C5)alkyl or (C1—C5)alkoxy or nitro or phenyl; in the presence of a monoesterification reagent selected from the group of mineral acids, alkanesulfonic acids, arylsulfonic acids, Lewis acids, and phosphorus pentoxide, to give a 1-(2-protected amino-4-thiazolyl)propene-1,3-dicarboxylic acid monoester (VI) according to the reaction scheme:

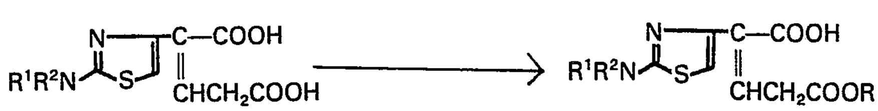

(V) (VI)

In another aspect, the invention provides a process of producing aminothiazolylpropenedicarboxylic acid monoesters, characterized by the step of: reacting a 1-(2-protected amino-4-thiazolyl)propene-1,3-dicarboxylic acid (V) with an alcohol selected from (C1—C5)alkanol; (C2—C8)alkenol; (C1—C5)alkanol substituted by halogen or (C1—C5)alkoxy or sulfonyl; benzylalcohol; or benzylalcohol substituted by (C1—C5)alkyl or (C1—C5)alkoxy or nitro or phenyl; in the presence of monoesterification reagent selected from the group of mineral acids, alkane sulfonic acids, arylsulfonic acids, Lewis acids and phosphorus pentoxide, to give a 1-(2-protected amino-4-thiazolyl)propene-1,3-dicarboxylic acid monoester (VI) according to the reaction scheme:

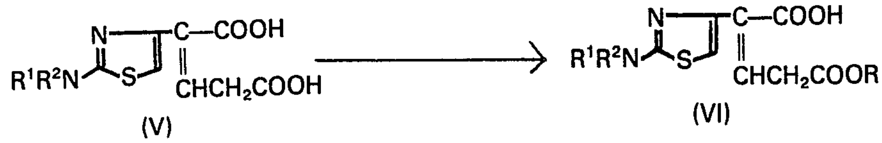

(V) (VI)

In the above formulae (I to VI), Hal is a halogen atom, R is the same or different ester-forming group selected from (C1—C5)alkyl; (C2—C8)alkenyl; (C1—C5)alkyl substituted by halogen or (C1—C5)alkoxy or sulfonyl; benzyl; benzyl substituted by (C1—C5)alkyl or (C1—C5)alkoxy or nitro or phenyl; $R^1$ is an amino-protecting group selected from (C1—C5)alkanoyl; halo(C1—C5)alkanoyl; (C1—C5)alkoxycarbonyl; (C8—C15)aralkoxycarbonyl; (C13—C20)polyarylmethyl or tri-(C1—C5)alkylsilyl; $R^2$ is hydrogen and $R^3$ is hydrogen or a group COOR.

The total 5-step process can be illustrated by the following reaction scheme wherein R, $R^1$, $R^2$, $R^3$ and Hal have the afore-said meanings. The last step of this process (i.e. the mono-esterification step) is the most important therein. The intermediates of formulae (II), (III), (IV) and (V) are novel compounds.

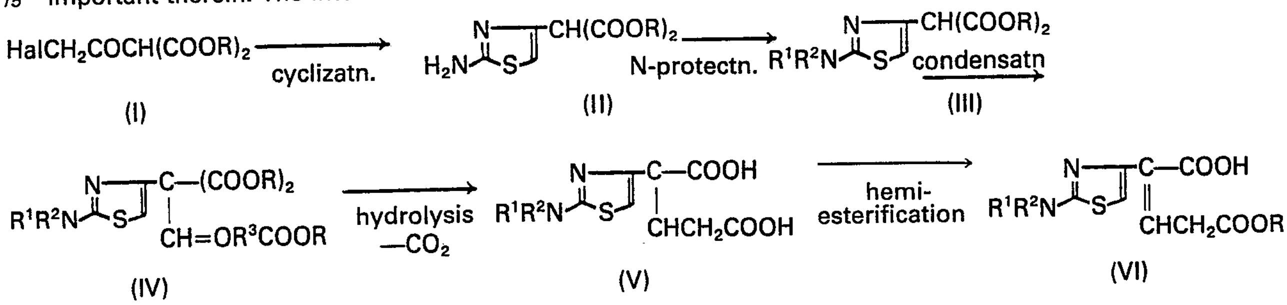


(I) (II) (III)

(IV) (V) (VI)

The individual steps of the process will now be described more in detail.

Step 1. Cyclization.

The reaction of a 2-haloacetylmalonic acid diester (I) with thiourea (1 to 5 molar equivalents) in a polar organic solvent gives a 2-(2-amino-4-thiazolyl)malonic acid diester (II) in good yield. This reaction proceeds well even at room temperature and can be completed within 1 to 15 hours. The product is in the form of a hydrochloride. The free amine can be recovered by neutralization.

Step 2. N-Protection.

Protecting the amino group of a compound (II) by a conventional method gives the corresponding compound (III).

Representative protection methods are: acylation with a halide or anhydride of the corresponding acid in the presence of a base (e.g. pyridine); tritylation with the corresponding halide in the presence of a hydrogen halide trapping agent; and enamine formation with the corresponding carbonyl compound in the presence of an acid.

Step 3. Condensation.

For the condensation reaction, a compound (III) is first treated with a strong base (e.g. a hydride, lower alkoxide or hydroxide of lithium, sodium or patassium) (1 to 5 molar equivalents) in an aprotic solvent (e.g. ether, tetrahydrofuran, or dioxane) to give the corresponding metal compound, and then the product is treated *in situ* with a halo (e.g. chloro or bromo)alkenoic acid ester (1 to 3 molar equivalents) for 0.5 to 12 hours at 0 to 50°C to give the corresponding (tri or tetra)carboxylic acid tri- or tetra-ester (IV).

Step 4. Hydrolysis and decarboxylation.

When the ester groups of compound (IV) are removed to give free carboxyl groups, one of the two carboxyl groups bound to a single carbon atom is eliminated as carbon dioxide at −10° to 50°C. This results into a dicarboxylic acid (V).

The removal of the ester groups can be effected by example by means of the following conventional reactions;

1) alkyl esters can be hydrolyzed with an excess of a base (e.g., alkali metal hydroxide) at −10° to 50°C for 0.5 to 10 hours to give free carboxylic acids;

2) highly reactive esters can be deprotected in an aqueous solution (e.g., by contacting them with an acid, a base, a buffer or an ion-exchange resin). When the reactivity is low, such reactivity can be elevated in a conventional way, e.g. in the case of a trichloroethyl ester by reaction with metal and acid; in the case or a p-nitrobenzyl ester by catalytic reduction of reaction with a dithionate salt; and in the case of phenacyl esters by irradiation;

3) aralkyl esters can be deprotected conventionally by reduction with hydrogen in the presence of a catalyst (e.g. platinum, palladium, nickel);

4) aralkyl esters, cyclopropylmethyl esters and sulfonylethyl esters can be deprotected by solvolysis. This reaction is carried out with mineral acids, Lewis acids (e.g. aluminium chloride, tin chloride, titanium tetrachloride), sulfonic acids (e.g., methanesulfonic acid, trifluoromethanesulfonic acid) or strong carboxylic acids (e.g. trifluoroacetic acid), and if required in the presence of a cation scavenger;

5) phenacyl esters, alkenyl esters and hydroxyaralkyl esters can be deprotected with a base or a nucleophilic reagents. Photochemically reactive phenacyl esters can be deprotected by irradiation with light;

6) 2-alkenyl esters can be deprotected with an alkalimetal alkanoate and a palladium-triphenylphosphine complex giving the corresponding alkalimetal salt.

During these reactions or during working-up procedures said decarboxylation will proceed under neutral or acid conditions. Thus, conventional work-up procedures give the objective dicarboxylic acid in good yield.

Step 5. Mono-esterification.

When esterified in a conventional way, dicarboxylic acids (V) give diesters.

However, mono-esters (VI) can be produced in good yields by the action of an alcohol and a mono-esterification reagent on such dicarboxylic acids.

For example, the dicarboxylic acid (V) is dissolved in an aprotic inert solvent (e.g., a hydrocarbon or halohydrocarbon solvent) and mixed with an alcohol (to be esterified) and with a mono-esterification reagent which is selected from mineral acids (e.g. hydrochloric acid, sulfuric acid, phosphoric acid, perchloric acid), Lewis acids (e.g. phosphorus halide, thionyl halide, silyl halide, tin halide, titanium halide), sulfonic acids (e.g. methanesulfonic acid, ethanesulfonic acid, trifluoromethanesulfonic acid, benzenesulfonic acid, toluenesulfonic acid, bromobenzenesulfonic acid), and dehydrating reagents (e.g. phosphorus pentoxide). Thereupon the reaction is effected, preferably in the absence of a base, at −10° to 80°C (especially −5° to 40°C) for 0.3 hours to 12 days. Only the carboxyl group in 3-position is esterified then, giving the desired mono-ester (VI) in good yield.

All the afore-said synthetic methods are carried out usually at −70° to 100°C (especially −20° to 50°C) for 10 minutes to 10 hours. This can conveniently be done in a solvent and if required under dry conditions.

The reaction solvent can be selected from hydrocarbons (e.g. pentane, hexane, octane, benzene, toluene, xylene), halohydrocarbons (e.g. dichloromethane, chloroform, carbon tetrachloride, dichloroethane, trichloroethane, chlorobenzene), ethers (e.g. diethyl ether, methyl isobutyl ether, anisole, dioxane, tetrahydrofuran), ketones (e.g. acetone, methyl ethyl ketone, cyclohexanone), esters (e.g. ethyl acetate, isobutyl acetate, methyl benzoate), nitrohydrocarbons (e.g. nitromethane, nitrobenzene), nitriles (e.g. acetonitrile, benzonitrile), amides (e.g. formamide, acetamide, dimethylformamide, dimethylacetamide, hexamethylphosphorotriamide), sulfoxides (e.g. dimethyl sulfoxide), carboxylic acids (e.g. formic acid, acetic acid, propionic acid), organic bases (e.g. diethylamine, triethylamine, pyridine, picoline, collidine, quinoline), alcohols (e.g. methanol, ethanol, propanol, hexanol, octanol, benzyl alcohol), water, or mixtures thereof. In some cases, an excess of reagent can serve as a solvent for the reaction.

The objective products can be obtained from their reaction mixtures by removing contaminants (e.g. unreacted starting materials, by-products, solvents) with conventional methods (e.g. extraction, evaporation, washing, concentration, precipitation, filtration, drying) and are worked up conventionally (e.g. by adsorption, elution, distillation, precipitation, separation, chromatography).

The following Examples illustrate the embodiments of this invention.

Physical constants of the products are listed in Tables 4 to 7. In the tables, IR spectra have been represented by wave numbers in $cm^{-1}$ and NMR spectra by delta values in ppm and J values in Hz.

The amounts are given in parts (by weight) or in (molar) equivalents based on the starting materials. All concentration steps during working-up procedures are effected under reduced pressure. The end products are identified by comparing their physical constants with those of compounds prepared by an alternative route.

The abbreviations used in the examples and tables are as follows:

BOC = tertiary-butoxycarbonyl,
Et = ethyl,
Ms = methanesulfonyl,
THF = tetrahydrofuran,
Cbz = benzyloxycarbonyl,
Me = methyl,
Ph = phenyl,
rt = room temperature.

Example 1

$ClCH_2COCH(COOEt)_2$

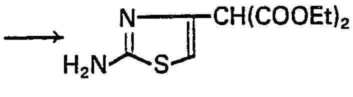

To a solution of 2-(chloroacetyl)malonic acid diethyl ester in ethanol (8 parts) is added thiourea (1.9 equivalents). After stirring at room temperature for 2 hours, the mixture is allowed to stand overnight. The reaction mixture is concentrated, diluted with water, and neutralized with aqueous sodium hydrogen carbonate to give 2-(2-amino-4-thiazolyl)malonic acid diethyl ester. Yield: 79.2%. m.p. 99.5—100.5°C.

IR($CHCl_3$) ν: 3475, 3390, 2970, 1925 $cm^{-1}$

NMR($CDCl_3$) δ: 1.25 (t, J=6.6Hz, 6H), 4.23 (q, J=6.6Hz, 4H), 4.65 (s, 1H), 5.64 (brs, 2H), 6.54 (s, 1H).

Example 2-1

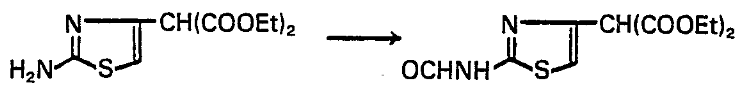

A solution of 2-(2-amino-4-thiazolyl)malonic acid diethyl ester in a mixture of acetic anhydride (6 parts) and formic acid (6 parts) is stirred at 0°C for 1 hour. The reaction mixture is concentrated in vacuum, diluted with ethyl acetate, washed with diluted hydrochloric acid, aqueous sodium hydrogen carbonate, and water, dried, and concentrated in vacuum to give 2-(2-formamido-4-thiazolyl)malonic acid diethyl ester. Yield: 70%.

Example 2-2

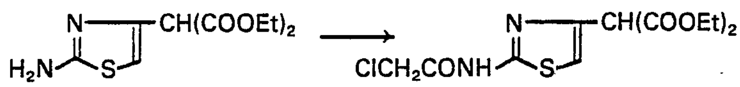

To a solution of 2-(2-amino-4-thiazolyl)malonic acid diethyl ester in N,N-dimethylformamide (10 parts) is added monochloroacetyl chloride (1.3 equivalents) under ice cooling. After stirring under ice cooling for 30 minutes and at room temperature for 1 hour, the reaction mixture is diluted with ethyl acetate and water, washed with water, dried, and concentrated in vacuum to give 2-(2-monochloroacetamido-4-thiazolyl)-malonic acid diethyl ester. Yield: 99.6%.

Example 2-3

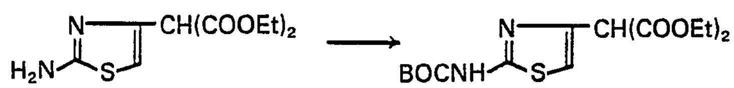

A solution of 2-(2-amino-4-thiazolyl)malonic acid diethyl ester in t-butyl pyrocarbonate (2.25 equivalents) is stirred overnight at 80°C. The mixture is diluted with ethyl acetate, washed with diluted hydrochloric acid, aqueous sodium hydrogen carbonate, and water, dired and concentrated under vacuum to give 2-(2-t-butoxycarbonylamino-4-thiazolyl)malonic acid diethyl ester. Yield: 73.3%.

Example 2-4

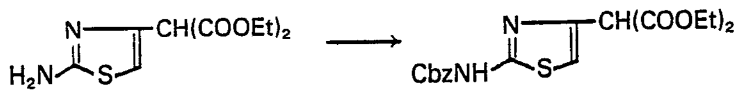

To a solution of 2-(2-amino-4-thiazolyl)malonic acid diethyl ester in dichloromethane (24 parts) are added pyridine (2 equivalents) and benzyl chloroformate (2 equivalents) at 0°C. After stirring for 30 minutes, the reaction mixture is washed with water, dried, and concentrated in vacuum to give 2-(2-benzyloxycarbonylamino-4-thiazolyl)malonic acid diethyl ester. Yield: 71.3%.

Example 2-5

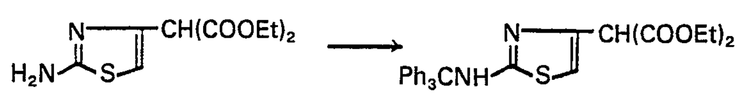

To a solution of 2-(2-amino-4-thiazolyl)malonic acid diethyl ester in dichloromethane (15 parts) are added trityl chloride (1.05 equivalents) and triethylamine (1.05 equivalents) under nitrogen at 0°C. After stirring at room temperature for 24 hours, the reaction mixture is poured into diluted hydrochloric acid and extracted with ethyl acetate. The extract is washed with water, dried, and concentrated in vacuum to give 2-(2-tritylamino-4-thiazolyl)malonic acid diethyl ester. Yield: 98.2%.

Example 3-1

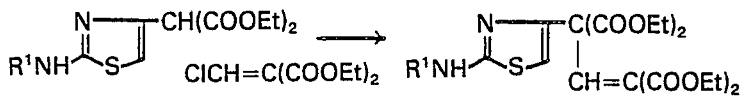

(3) (4)

To a solution of a 2-(2-protected amino-4-thiazolyl)malonic acid diethyl ester in tetrahydrofuran (5 to 40 parts) is added sodium hydride (2 to 3 equivalents). After stirring at 0°C for 5 to 30 minutes, the produced solution of the 2-sodiomalonate is mixed with chloromethylidenemalonic acid diethyl ester (1 to 2 equivalents). After stirring at room temperature for 1 to 10 hours, the reaction mixture is treated with diluted hydrochloric acid and ethyl acetate. The organic layer is separated, washed with aqueous sodium hydrogen carbonate and water, dried, concetrated, and purified by chromatography to give 1-(2-protected amino-4-thiazolyl)propene-1,1,3,3-tetracarboxylic acid tetraethyl ester. Yield: 10 to 70%.

The reaction conditions are listed in Table 1.

Example 3-2

If Example 3-1 is repeated with substitution of tetrahydrofuran by dioxane or dimethoxyethane, the objective tetracarboxylic acid ester can be produced in nearly the same yield.

Example 4-1

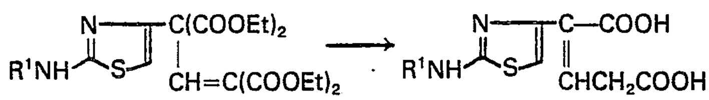

(4) (5)

To a solution of a 1-(2-protected amino-4-thiazolyl)propene-1,1,3,3-tetracarboxylic acid tetraethyl ester in tetrahydrofuran (2 to 10 parts) are added water (5 to 30 parts) and sodium hydroxide (1 to 30 equivalents). After stirring at room temperature for 30 minutes to 10 hours, the reaction mixture is washed with ethyl acetate, acidified with hydrochloric acid, and extracted with ethyl acetate. The extract solution is washed with water, dried, and concentrated to give 1-(2-protected amino-4-thiazolyl)propene-1,3-dicarboxylic acid. Yield: 50 to 75%.

The reaction conditions are listed in Table 2.

Example 4-2

The same dicarboxylic acid is formed under the same conditions wherein the solvent of Example 4-1 is substituted by methanol or ethanol.

Example 5

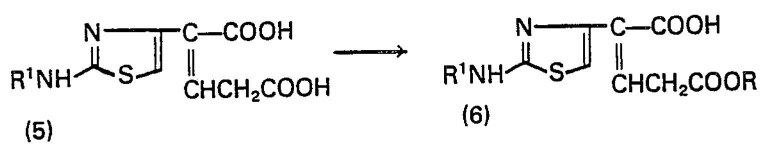

(5) (6)

A mixture of a 1-(2-protected amino-4-thiazolyl)propene-1,3-dicarboxylic acid in a solvent (5 to 30 parts), with alcohol (4 to 10 equivalents), and a catalyst (1 to 10 equivalents) is allowed to react for 1 to 100 hours at 0 to 40°C. The mixture is diluted with ethyl acetate and water, washed with water, aqueous sodium hydrogen carbonate, and hydrochloric acid, dried, and concentrated. The residue is crystallized from methanol to give the corresponding mono-ester. Yield: 50 to 90%.

The reaction conditions are listed in Table 3.

Example 6

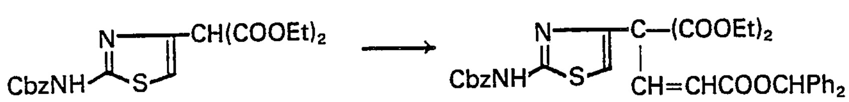

To a solution of 2-(2-benzyloxycarbonylamino-4-thiazolyl)malonic acid diethyl ester in tetrahydrofuran (20 parts) are added sodium hydride (2.35 equivalents) and a solution of trans-3-chloroacrylic acid diphenylmethyl ester (1.2 equivalents) in tetrahydrofuran (8 parts) under ice cooling. After stirring at 35 to 40°C for 12.5 hours, the mixture is diluted with ethyl acetate and diluted hydrochloric acid, washed with water, dried, and concentrated. The residue is chromatographed over silica gel. The fraction eluted with a

mixture of benzene and ethyl acetate (15:1) is crystallized from ethyl acetate to give 3-diphenylmethoxycarbonyl-1-(2-benzyloxycarbonylamino-4-thiazolyl)-2-propene-1,1-dicarboxylic acid diethyl ester.

IR($CHCl_3$) ν: 3390, 2960, 1730, 1635 $cm^{-1}$.

NMR($CDCl_3$) δ: 1.73 (t, J=6.6Hz, 6H), 4.25 (q, J=6.6Hz, 4H), 5.25 (s, 2H), 5.81 (d, J=15.8Hz, 1H), 6.93 (s, 1H), 7.25 ~ 7.45 (m, 16H), 7.60 (d, J=15.8Hz, 1H), 8.07 (brs, 1H).

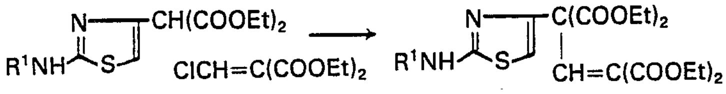


(3) (4)

| $R^1$ | CHO | $ClCH_2CO$ | BOC | Cbz | $Ph_3C$ |
|---|---|---|---|---|---|
| THF(parts) | 22 | 20 | 23 | 18 | 18 |
| NaH (equiv.) | 2.48 | 2.39 | 2.43 | 2.46 | 2.50 |
| Na-salt(min.) | 15 | 20 | 20 | 10 | 15 |
| reagent (equiv.) | 1.46 | 1.30 | 1.14 | 1.40 | 1.30 |
| condensation (hours) | 1 | 2 | 3 | 1.5 | 5 |
| yield (%) | 32.7 | 62.1 | 43.2 | 67.4 | 13 |

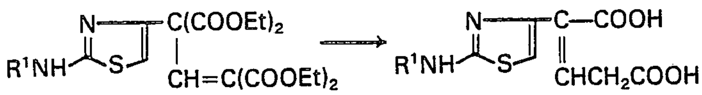

(4) (5)

| R[1] | CHO | $ClCH_2CO$ | BOC | Cbz | Cbz/R=Me |
|---|---|---|---|---|---|
| THF (parts) | 5.26 | 3.53 | 2.29 | 6.40 | 5.73 |
| NaOH (equiv.) | 24 | 12.5 | 12.5 | 10 | 5.15 |
| water (parts) | 26 | 11.8 | 11.5 | 20 | − |
| hydrolysis (min.) | 300 | 40 | 180 | 180 | 90 |
| yield (%) | 8 | 46.6 | 73.8 | 47.9 | 81.3 |

TABLE 3 (Part 1) Conditions of mono-esterification

(TMSC = $Me_3SiCl$)

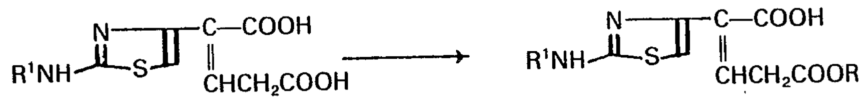

(5) (6)

| R' | CHO | CHO | $ClCH_2CO$ | $ClCH_2CO$ | BOC | Cbz | | |
|---|---|---|---|---|---|---|---|---|
| R | $PhCH_2$ | $PhCH_2$ | $PhCH_2$ | $CH_3$ | $PhCH_2$ | Me | Me | $t-C_4H_9$ |
| ROH (equiv.) | 9.0 | 9.28 | 10.7 | 10 | 10 | 27 part | 15 part | 10 |
| solvent (parts) | $CH_2Cl_2$ 11.2 | $CH_2Cl_2$ 13.2 | $CH_2Cl_2$ 10.9 | $CH_2Cl_2$ 7.8 | $CH_2Cl_2$ 13.8 | $CH_3OH$ 27.5 | $CH_2Cl_2$ 15 | $CH_2Cl_2$ 8.28 |
| catalyst (equiv.) | $PCl_5$ 1.0 | TMSC 2.6 | $SOCl_2$ 1.50 | $SOCl_2$ 1.2 | $SOCl_2$ 1.2 | $TiCl_4$ 1.8 | HCl 7.2 | $P_2O_5$ 1.0 |
| temperature | rt | rt | rt | rt | rt | 0°C | rt | rt |
| time (hr) | 1.5 | 1.5 | 1.5 | 1.5 | 1.5 | 0.3 | 0.5 | 20 |
| yield (%) | 65 | 61 | 81 | 96 | 70 | 100 | 90 | 16 |

TABLE 3 (Part 2) Conditions of mono-esterification

| $R^1$ | Cbz | | | | | | |
|---|---|---|---|---|---|---|---|
| R | $PhCH_2$ | $PhCH_2$ | $PhCH_2$ | $PhCH_2$ | $PhCH_2$ | $PhCH_2$ | p-$MePhCH_2$ |
| ROH (equiv.) | 9.63 | 4.82 | 5 | 9.63 | 9.3 | 9.63 | 10 |
| solvent (parts) | $CH_2Cl_2$ 7.32 | $CH_2Cl_2$ 9.14 | $CH_2Cl_2$ 5.5 | $CH_2Cl_2$ 7.32 | $CH_2Cl_2$ 2.76 | $CH_2Cl_2$ 11.0 | $CH_2Cl_2$ 20 |
| catalyst (equiv.) | HCl 7.2 | $PCl_5$ 1.10 | $POCl_3$ 1.3 | $SOCl_2$ 1.20 | $TiCl_4$ 2.0 | TMSC 2.6 | $SOCl_2$ 1.2 |
| temperature | rt | rt | rt | rt | rt | rt | rt |
| time (hr) | 96 | 7 | 16 | 2 | 3.5 | 3 | 3 |
| yield (%) | 59 | 73 | 86 | 83 | 60 | 80 | 80 |

TABLE 3 (Part 3) Conditions of mono-esterification

| $R^1$ | Cbz | | | | | |
|---|---|---|---|---|---|---|
| R | p-MeOPhCH$_2$ | CH$_2$=CHCH$_2$ | CH$_2$=CHCH$_2$ | MeCH=CHCH$_2$ | CH$_2$=CMe-CH$_2$ | CH$_2$=CHCHMe |
| ROH (equiv.) | 5 | 10 | 10 | 10 | 5 | 5 |
| solvent (parts) | $CH_2Cl_2$ 5 | $CH_2Cl_2$ 13.8 | $CH_2Cl_2$ 13.8 | $CH_2Cl_2$ 13.8 | $CH_2Cl_2$ 13.8 | $CH_2Cl_2$ 13.8 |
| catalyst (equiv.) | MsOH 1.2 | $SOCl_2$ 1.2 | MsOH 1.3 | $SOCl_2$ 1.2 | MsOH 1.3 | MsOH 1.3 |
| temperature | 0~5°C | 0°C~rt | 0~5°C | rt | 0~5°C | 0~5°C |
| time (hr) | 120 | 5 | 20 | 24 | 240 | 144 |
| yield (%) | 43 | 88 | 90 | 88 | 86 | 88 |

TABLE 3 (Part 4) Conditions of mono-esterification

| R' | Cbz | | |
|---|---|---|---|
| R | $Me_2C=CHCH_2$ | $Me_2C=CHCH_2$ *) | $PhCH=CHCH_2$ |
| ROH (equiv.) | 5 | 5 | 5 |
| solvent (parts) | $CH_2Cl_2$ 8.3 | $CH_2Cl_2$ 13.8 | $CH_2Cl_2$ 13.8 |
| catalyst (equiv.) | MsOH 1.3 | MsOH 1.3 | MsOH 1.3 |
| temperature | 0~5°C | rt | 0~5°C |
| time (hr) | 168 | 96 | 72 |
| yield (%) | 75 | 44 | 71 |

*) $CH_2=CHCMe_2OH$ is used as ROH

TABLE 4

Physical constants of 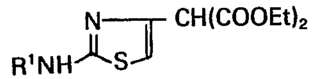 (3)

| R' | IR($CHCl_3$) $\nu$ : $cm^{-1}$ | NMR($CDCl_3$) $\delta$ : ppm |
|---|---|---|
| CHO | 3380, 3160, 1740, 1725, 1685, mp73° | 1.23(t, J=7Hz, 6H), 4.20(q, J=7Hz, 4H), 4.77(s, 1H), 7.06(s, 1H), 8.72(s, 1H), 11.65(brs, 1H). |
| $ClCH_2CO$ | 3500, 2950, 1750, 1725, 1680. | 1.25(t, J=7.5Hz, 6H), 4.23(q, J=7.5Hz, 4H), 4.27(s, 2H), 4.73(s, 1H), 7.10(s, 1H), 9.65(br, 1H). |
| BOC | 3420, 1750, 1730 ($CCl_4$). | 1.23(t, J=7Hz, 6H), 1.54(s, 9H), 4.20(q, J=7Hz, 4H), 4.89(s, 1H), 6.99(s, 1H), 9.10(brs, 1H). |
| Cbz | 3380, 3140, 2950, 1725, mp. 80 °C | 1.22(t, J=6.9Hz, 6H), 4.16(q, J=6.9Hz, 4H), 5.08(s, 1H), 5.29(s, 2H), 7.04(s, 1H), 7.2~7.5(m, 5H). |
| $Ph_3C$ | 3615, 3405, 2975, 1730. | 1.24(t, J=7.2Hz, 6H), 4.18(q, J=7.2Hz, 4H), 4.65(s, 1H), 6.40(s, 1H), 6.61 (brs, 1H), 7.29(brs, 15H). |

TABLE 5

**Physical constants of** 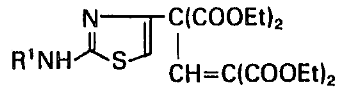 (4)

| $R^1$ | IR($CHCl_3$) $\nu$ : $cm^{-1}$ | NMR($CDCl_3$) $\delta$ : ppm |
|---|---|---|
| CHO | 3380, 3150, 2970, 1745, 1710(sh). | 1.11~1.37(m, 12H), 4.12(q, J=7.5Hz, 2H), 4.20(q, J=7.5Hz, 4H), 4.28(q, J=7.5 Hz, 2H), 7.41(s, 1H), 7.70(s, 1H), 8.65(brs, 1H). |
| $ClCH_2CO$ | 3375, 2980, 1740, 1720, 1690, 1650. | 1.17, 1.23, 1.31(3×t, J=8Hz, 12H), 3.85~4.32(m, 8H), 4.21(s, 2H), 7.45(s, 1H), 7.61(s, 1H), 10.12(brs, 1H). |
| BOC | 3400, 2965, 1730, 1635. mp. 126°C . | 1.10~1.38(m, 12H), 4.07(q, J=6.7Hz, 2H), 4.25(q, J=6.7Hz, 2H), 4.26(q, J=6.7 Hz, 2H), 7.42(s, 1H), 7.78(s, 1H), 7.97(brs, 1H). |
| Cbz | 3380, 2950, 1730, 1640. | 1.10~1.40(m, 12H), 4.02(q, J=7.5Hz, 2H), 4.20(q, J=7.5Hz, 6H), 5.21(s, 2H), 7.34(s, 5H), 7.77(s, 1H). |
| $Ph_3C$ | 1730, 1635. | 1.05~1.40(m, 12H), 3.96(q, J=7.5Hz, 2H), 4.15(q, J=7.5Hz, 2H), 4.25(q, J=7.5 Hz, 2H), 6.48(brs, 1H), 6.85(s, 1H), 7.25(brs, 15H), 7.75(s, 1H). |
| H | 1730, 1595. mp. 113~114°C | 1.1~1.4(m, 12H), 3.95~4.4(m, 8H), 5.41(brs, 2H), 7.02(s, 1H), 7.75(s, 1H). |

TABLE 6

Physical constants of 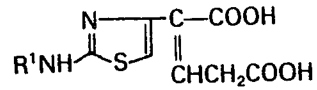 (5)

| R' | IR(Nujol) ν : $cm^{-1}$ | NMR($CD_3SOCD_3$) δ : ppm |
|---|---|---|
| BOC cis | 3120, 1700, 1675. dp 153~154°C | 1.50(s, 9H), 3.45(d, J=7.5Hz, 2H), 7.00(t, J=7.5Hz, 1H), 7.13(s, 1H). |
| BOC trans | 3150, 1700, 1630, 1600. dp 165~167°C. | 1.49(s, 9H), 3.41(d, J=7.5Hz, 2H), 6.89(t, J=7.5Hz, 1H), 7.08(s, 1H). |
| Cbz | 3200, 1738, 1715, 1690. dp 169~172°C. | 3.44, 3.50, (2×d, J=8Hz, 2H), 5.25(s, 2H), 7.07, 7.35(2×t, J=8Hz, 1H), 7.12(s, 1H), 7.38(brs, 5H) [$CDCl_3$+$CD_3OD$] |
| HCO | 3400, 1718, 1690, 1630, 1550. dp 168°C. | 3.45, 3.63(2×d, J=7.5Hz, 2H), 7.14, 7.32(2×t, J=7.5Hz, 1H), 7.23, 7.25(2×s, 1H), 8.51(s, 1H) [$CDCl_3$+$CD_3OD$]. |
| $ClCH_2CO$- | 3100, 1720, 1685, 1620. dp 153~155°C. | 3.45(d, J=8Hz, 2H), 4.37(s, 2H), 6.97, 7.05(2×t, J=8Hz, 1H), 7.23, 7.27(2×s, 1H). |

TABLE 7 (Part 1)

Physical constants of 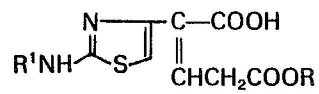 (6)

| R' | R | IR(Nujol) ν : cm⁻¹ | NMR($CD_3SOCD_3$) δ : ppm |
|---|---|---|---|
| BOC | t-Bu | 1725, 1620, 1545 [$CHCl_3$] | 1.45(s, 9H), 1.53(s, 9H), 3.30, 3.64(d+d, J=7Hz, 2H), 6.98, 7.04(s+s, 1H), 6.99, 7.32(t+t, J=7Hz, 1H) [$CDCl_3$]. |
| BOC | Bzl | 3160, 1740, 1724, 1700, 1678, 1255, 1168. | 3.95(d, J=7.5Hz, 2H), 5.50(s, 2H), 7.26(t, J=7.5Hz, 1H), 7.30(brs, 1H), 7,49(s, 1H), 7.75(s, 5H), 11.86(brs, 1H). |
| HCO (1 cis: 2 trans) | t-Bu | 3150, 3100, 1720, 1690, 1635. mp. 185~188°C | 1.40(s, 9H), 3.43(d, J=7Hz, 2H), 6.89, 7.00(2×t, J=7Hz, 1H), 7.20, 7.26 (2×s, 1H), 8.48(s, 1H). |
| HCO | Bzl | 1735, 1680, 1620. dp 153~155°C. | 3.69(d, J=7Hz, 2H), 5.12(s, 2H), 7.17(t, J=7Hz, 1H), 7.21(s, 1H), 7.32 (s, 5H), 8.46(s, 1H). |
| $ClCH_2CO$ | Me | nd | 3.39(d, J=7.5Hz, 2H), 3.70(s, 3H), 4.24(s, 2H), 7.11(s, 1H), 7.23(t, J=7.5Hz, 1H), 9.37(brs, 2H) [$CDCl_3$]. |
| $ClCH_2CO$ | Bzl | 1726, 1685, 1160. dp 155°C. | 3.95, 4.01(2×d, J=7.5Hz, 2H), 4.71(s, 2H), 5.45, 5.47(2×s, 2H), 7.28, 7.40(2×t, J=7.5Hz, 1H), 7.58, 7.65(2×s, 1H), 7.70(s, 5H), 12.9(brs, 1H). |

TABLE 7 (Part 2)

| R' | R | IR(Nujol) ν : $cm^{-1}$ | NMR($CD_3SOCD_3$) δ : ppm |
|---|---|---|---|
| Cbz | Me | 3400~2300, 1740, 1550. | 3.58~3.73(m, 2H), 3.63(s, 3H), 5.27(s, 2H), 7.03~7.46(m, 7H). |
| Cbz (trans) | t-Bu | 3160~2200, 1720, 1680, 1635. mp. 169~171°C | 1.42(s, 9H), 3.53(d, J=7Hz, 2H), 5.29(s, 1H), 7.27(t, J=7Hz, 1H), 7.35 (s, 1H), 7.30~7.50(m, 5H). |
| Cbz (cis) | t-Bu | nd | 1.44(s, 9H), 3.53(d, J=7Hz, 2H), 5.27(s, 2H), 7.13(t, J=7Hz, 1H), 7.24 (s, 1H), 7.30~7.47(m, 5H) [$CDCl_3$]. |
| Cbz (2 cis: 1 trans) | Me-Bzl | 3150~2050, 1720, 1670, 1620, 1570. mp. 160~163°C. | 2.33(s, 3H), 2.53, 2.70(2×d, J=8Hz, 2H), 5.11(s, 2H), 5.26(s, 2H), 6.99~7.40(m, 10H) [$CDCl_3$-$CD_3OD$]. |
| Cbz (2 cis: 3 trans) | Bzl | 1725, 1675, 1620, 1575. mp. 164~166°C. | 3.51, 3.73(2×d, J=7Hz, 2H), 5.13(s, 2H), 5.26(s, 2H), 7.06, 7.10(2×s, 1H), 7.0~7.5(m, 11H)[$CDCl_3$-$CD_3OD$]. |
| Cbz | PMB | 1720, 1575, 1515. mp. 145~148°C. | 3.80(d, J=8Hz, 2H), 3.90(s, 3H), 5.20(s, 2H), 5.33(s, 2H), 7.00(s, 1H), 6.85~7.60(m, 10H) [$CDCl_3$-$CD_3OD$]. |
| Cbz (1 cis: 2 trans) | $CH_2$<br>CH<br>$CH_2$ | 3515, 2480(br), 1736, 1549, 1305, 1086 ($CHCl_3$) mp. 122~130°C. | 3.35(d, J=8Hz, 4/3H), 3.68(d, J=8Hz, 2/3H), 4.56(d, J=6Hz, 2H), 5.11~5.37(m, 4H), 5.65~6.15(m, 1H), 6.90~7.41(m, 7H), 9.82(bs, 2H).[$CDCl_3$]. |

TABLE 7 (Part 3)

| R' | R | IR(Nujol) ν : $cm^{-1}$ | NMR($CD_3SOCD_3$) δ : ppm |
|---|---|---|---|
| Cbz (1 cis: 5 trans) | CHMe<br>CH<br>$CH_2$ | 3420, 2500(br), 1732, 1549, 1302, 1087 ($CHCl_3$)<br>mp. 127~131°C. | 1.16(d, J=7Hz, 1/2H), 1.29(d, J=7Hz, 5/2H), 3.46(d, J=8Hz, 5/3H), 3.68 (d, J=8Hz, 1/3H), 5.05~5.49(m, 3H), 5.16(s, 2H), 5.66~6.02(m, 1H), 7.08~7.57(m, 7H) [$CDCl_3$-$CD_3OD$]. |
| Cbz (9 cis: 11trans) | $CH_2$<br>CMe<br>$CH_2$ | 3420, 1736, 1548, 1307, 1085 ($CHCl_3$)<br>mp. 120~123°C. | 1.73(s, 3H), 3.52(d, J=8.5Hz, 11/10H), 3.73(d, J=8.5Hz, 9/10H), 4.54(s, 2H), 4.95(brs, 2H), 5.26(s, 2H), 6.99~7.46(m, 7H) [$CDCl_3$-$CD_3OD$]. |
| Cbz (1 cis: 4 trans) | $CH_2$<br>CH<br>CHMe | 3415, 1732, 1548, 1304, 1076 ($CHCl_3$)<br>mp. 139~142°C (decomp.). | 1.67(d, J=6Hz, 3H), 3.44(d, J=8Hz, 8/5H), 3.64(d, J=8Hz, 2/5H), 4.49(d, J=6Hz, 2H), 5.23(s, 2H), 5.35~6.05(m, 2H), 7.05~7.41(m, 7H) [$CDCl_3$-$CDCl_3$]. |
| Cbz (1 cis: 2 trans) | $CH_2$<br>CH<br>$CMe_2$ | 3175, 2520(br), 1732, 1659, 1071. mp. 167~168°C (decomp.). | 1.98(s, 3H), 2.03(s, 3H), 3.82(d, J=8Hz, 4/3H), 3.86(d, J=8Hz, 2/3H), 4.87(d, J=7Hz, 2H), 5.64(s, 2H), 5.52~5.71(m, 1H), 7.21(t, J=8Hz, 1/3H), 7.65~7.69(m, 5+2/3H) [$CD_3SOCD_3$-$CD_3OD$(external TMS)]. |
| Cbz | $CH_2$<br>CH<br>CHPh | nd | TLC[EtOAc/$CHCl_3$(1:1)]: Rf=0.2 |

**Claims**

1. A process of producing aminothiazolylpropenedicarboxylic acid mono-esters, characterized by the steps of:

1) reacting a haloacetylmalonic acid diester (I) with thiourea to give a 2-(2-amino-4-thiazolyl)malonic acid diester (II) according to the reaction scheme:

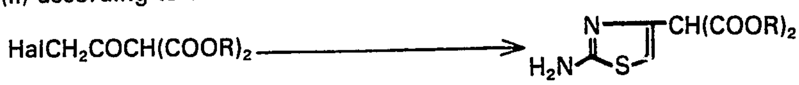

(I) (II)

wherein Hal is a halogen atom, and R is the same or different ester-forming group selected from (C1—C5)-alkyl; (C2—C8)alkenyl, (C1—C5)alkyl substituted by halogen or (C1—C5)alkoxy or sulfonyl; benzyl; benzyl substituted by (C1—C5)alkyl or (C1—C5)alkoxy or nitro or phenyl;

2) reacting the compound (II) with an amino-protecting reagent to give a 2-(2-protected amino-4-thiazolyl)malonic acid diester (III) according to the reaction scheme:

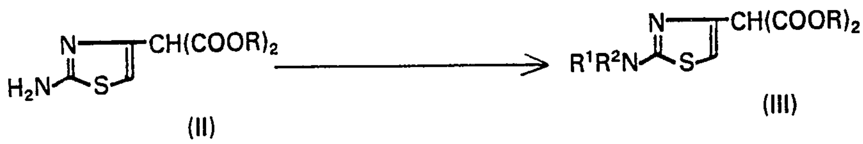

(II) (III)

wherein R has the afore-said meaning;

$R^1$ is an amino-protecting group selected from (C1—C5)alkanoyl; halo-(C1—C5)alkanoyl; (C1—C5)-alkoxycarbonyl; (C8—C15)aralkoxycarbonyl; (C13—C20)polyarylmethyl and tri-(C1—C5)alkylsilyl; and

$R^2$ is hydrogen;

3) reacting the hydrogen (III) with a haloalkenoic acid ester in an optic solvent and the presence of a strong base to give a 1-(2-protected amino-4-thiazolyl)-2-propene-(1,1,3-tri or 1,1,3,3-tetra)carboxylic acid tri- or tetra ester (IV), according to the reaction scheme:

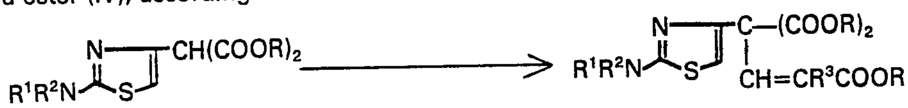

(III) (IV)

wherein Hal, R, $R^1$ and $R^2$ have the afore-said meanings and $R^3$ is hydrogen or a group COOR;

4) subjecting the compound (IV) to hydrolysis and decarboxylation to give a 1-(2-protected amino-4-thiazolyl)propene-1,3-dicarboxylic acid (V), according to the reaction scheme:

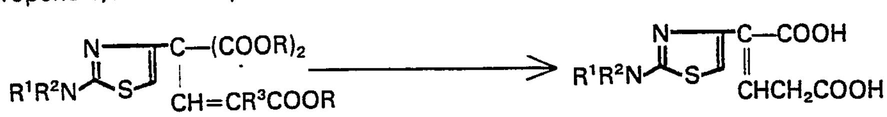

(IV) (V)

wherein R, $R^1$, $R^2$ and $R^3$ have the afore-said meanings;

5) reacting the compound (V) with an alcohol selected from a (C1—C5)alkanol; (C2—C8)alkenol; (C1—C5)alkanol substituted by halogen or (C1—C5)alkoxy or sulfonyl; benzylalcohol; or benzylalcohol substituted by (C1—C5)alkyl or (C1—C5)alkoxy or nitro or phenyl; in the presence of a monoesterification reagent selected from the group of mineral acids, alkanesulfonic acids, arylsulfonic acids, Lewis acids, and phosphorus pentoxide, to give a 1-(2-protected amino-4-thiazolyl)propene-1,3-dicarboxylic acid monoester (VI) according to the reaction scheme:

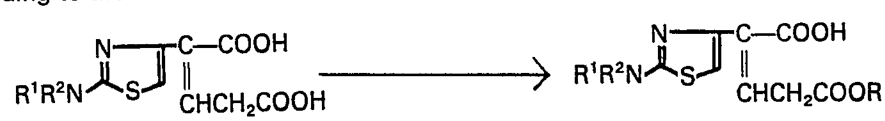

(V) (VI)

wherein $R^1$ and $R^2$ have the afore-said meanings and R is an ester forming group selected from (C1—C5)-alkyl; (C2—C8)alkenyl; (C1—C5)alkyl substituted by halogen or (C1—C5)alkoxy or sulfonyl; benzyl; or benzyl substituted by (C1—C5)alkyl or (C1—C5)alkoxy or nitro or phenyl.

2. A process of producing aminothiazolylpropenedicarboxylic acid monoesters, characterized by the step of reacting a 1-(2-protected amino-4-thiazolyl)propene-1,3-dicarboxylic acid (V) with an alcohol selected from (C1—C5)alkanol; (C2—C8)alkenol; (C1—C5)alkanol substituted by halogen or (C1—C5)alkoxy or sulfonyl; benzylalcohol; or benzylalcohol substituted by (C1—C5)alkyl or (C1—C5)alkoxy or nitro or phenyl; in the presence of a monoesterification reagent selected from the group of mineral acids, alkane sulfonic acids, arylsulfonic acids, Lewis acids, and phosphorus pentoxide, to give a 1-(2-protected amino-4-thiazolyl)propene-1,3-dicarboxylic acid monoester (VI) according to the reaction scheme:

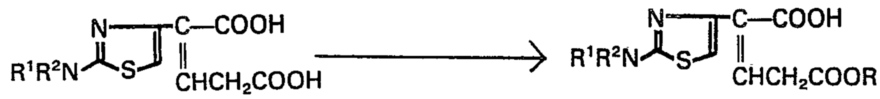

(V) (VI)

wherein R is an ester-forming group selected from (C1—C5)alkyl; (C2—C8)alkenyl; (C1—C5)alkyl substituted by halogen or (C1—C5)alkoxy or sulfonyl; benzyl; benzyl substituted by (C1—C5)alkyl or (C1—C5)alkoxy or nitro or phenyl;

$R^1$ is an amino-protecting group selected from (C1—C5)alkanoyl; halo-(C1—C5)alkanoyl; (C1—C5)-alkoxycarbonyl; (C8—C15)aralkoxycarbonyl and (C13—C20)polyarylmethyl or tri-(C1—C5)alkylsilyl; and

$R^2$ is hydrogen.

3. A process as claimed in claim 2, wherein the reaction is carried out in a solvent selected from hydrocarbons, halohydrocarbons, the reagent alcohol, and mixtures thereof.

4. A process as claimed in claim 2, wherein the reaction is carried out at −10° to 80°C for a period of 0.3 hours to 12 days.

5. Compounds represented by formulae VII and VIII

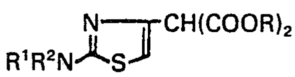

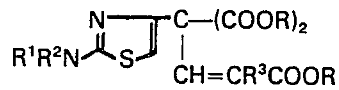

(VII) (VIII)

wherein R, $R^2$ and $R^3$ have the same meanings as in claim 1 or 2, and wherein $R^1$ may represent hydrogen or an amino-protecting group as specified in claim 1 or 2.

6. A compound as claimed in Claim 5, wherein R is (1C to 5C) alkyl, (1C to 5C) substituted alkyl, (2C to 8C) alkenyl, benzyl, or substituted benzyl.

7. A compound as claimed in Claim 5, wherein $R^1$ and $R^2$ each are hydrogen, (1C to 5C) alkanoyl, (1C to 5C) haloalkanoyl, (1C to 5C) alkoxycarbonyl, (8C to 15C) aralkoxycarbonyl, (13C to 20C) polyarylmethyl or tri(1C to 5C) alkylsilyl.

**Patentansprüche**

1. Verfahren zur Herstellung von Aminothiazolylpropendicarbonsäuremonoestern, gekennzeichnet durch die Stufen:

1) Umsetzung eines Haloacetylmalonsäurediesters (1) mit Thioharnstoff unter Bildung des 2-(2-Amino-4-thiazolyl)malonsäurediesters (II) nach dem Reaktionsschema:

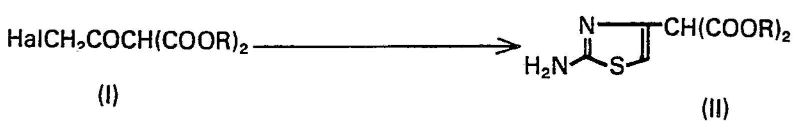

(I) (II)

worin Hal ein Halogenatom bedeutet und R die gleiche oder eine unterschiedliche esterbildende Gruppe, ausgewählt unter (C1—C5)-Alkyl, (C2—C8)-Alkenyl, (C1—C5)-Alkyl, substituiert durch Halogen, oder (C1—C5)-Alkoxy oder Sulfonyl, Benzyl, Benzyl, substituiert durch (C1—C5)-Alkyl oder (C1—C5)-Alkoxy oder Nitro oder Phenyl, bedeutet;

2) Umsetzung der Verbindung der Verbindung (II) mit einem Amino-Schutzreagens unter Bildung des 2-(2-geschützten-Amino-4-thiazolyl)malonsäurediesters (III) entsprechend dem Reaktionsschema:

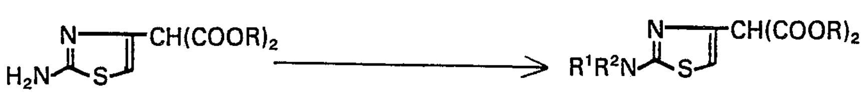

(II) (III)

worin R die zuvor gegebene Bedeutung besitzt,

$R^1$ eine Amino-Schutzgruppe, ausgewählt under (C1—C5)-Alkanoyl, Halo-(C1—C5)-alkanoyl, (C1—C5)-Alkoxycarbonyl, (C8—C15)-Aralkoxycarbonyl, (C13—C20)-Polyarylmethyl und Tri-(C1—C5)-alkylsilyl, bedeutet und

$R^2$ Wasserstoff bedeutet;

3) Umsetzung der Verbindung (III) mit einen Haloalkensäureester in einem aprotischen Lösungsmittel unter Anwesenheit einer starken Base unter Bildung des 1-(2-geschützten-Amino-4-thiazolyl)-2-propen-(1,1,3-tri- oder 1,1,3,3-tetra)carbonsäure-tri- oder tetraesters (IV) entsprechend dem Reaktionsschema

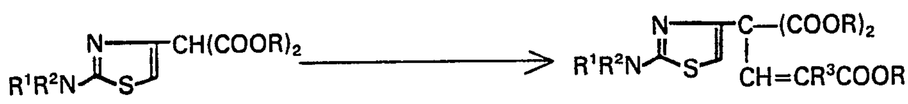

(III) (IV)

worin Hal, R, $R^1$ und $R^2$ die zuvor gegebenen Bedeutungen besitzen und $R^3$ Wasserstoff oder die Gruppe COOR bedeutet;

4) Unterwerfen der Verbindung (IV) der Hydrolyse und Decarboxylierung unter Bildung der 1-(2-geschützten-Amino-4-thiazolyl)propen-1,3-dicarbonsäure (V) entsprechend dem Reaktionsschema:

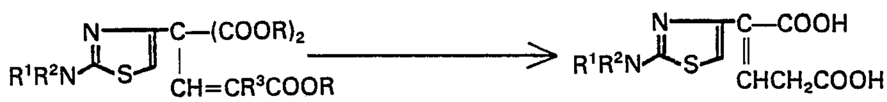

(IV) (V)

worin R, $R^1$, $R^2$ und $R^3$ die zuvor gegebenen Bedeutungen besitzen;

5) Umsetzung der Verbindung (V) mit einem Alkohol, ausgewählt unter (C1—C5)-Alkanol, (C2—C8)-Alkenol, (C1—C5)-Alkanol, substituiert durch Halogen oder (C1—C5)-Alkoxy oder Sulfonyl, Benzylalkohol oder Benzylalkohol, substituiert durch (C1—C5)-Alkyl oder (C1—C5)-Alkoxy oder Nitro oder Phenyl, in Anwesenheit eines Monoveresterungs-Reagenses, ausgewählt aus der Gruppe Mineralsäuren, Alkansulfonsäuren, Arylsulfonsäuren, Lewis-Säuren und Phosphorpentoxid, unter Bildung des 1-(2-geschützten-Amino-4-thiazolyl)-propen-1,3-dicarbonsäuremonoesters (VI) entsprechend dem Reatkionsschema:

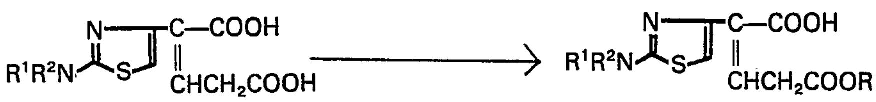

(V) (VI)

worin $R^1$ und $R^2$ die zuvor gegebenen Bedeutungen besitzen und R eine esterbildende Gruppe, ausgewählt under (C1—C5)-Alkyl, (C2—C8)-Alkenyl, (C1—C5)-Alkyl, substituiert durch Halogen oder (C1—C5)-Alkoxy oder Sulfonyl, Bebnzyl oder Benzyl, substituiert durch (C1—C5)-Alkyl oder (C1—C5)-Alkoxy oder Nitro oder Phenyl, bedeutet.

2. Verfahren zur Herstellung von Aminothiazolylpropendicarbonsäuremonoester, gekennzeichnet durch die Stufen: umsetzung einer 1-(2-geschützten-Amino-4-thiazolyl)-propen-1,3-dicarbonsäure (V) mit einem Alkohol, ausgewählt unter (C1—C5)-Alkanol, (C2—C8)-Alkenol, (C1—C5)-Alkanol, substituiert durch Halogen oder (C1—C5)-Alkoxy oder Sulfonyl, Benzylalkohol oder Benzylalkohol, substituiert durch (C1—C5)-Alkyl oder (C1—C5)-Alkoxy oder Nitro oder Phenyl, in Anwesenheit eines Monoveresterungs-Reagenses, ausgewählt aus der Gruppe Mineralsäuren, Alkansulfonsäuren, Arylsulfonsäuren, Lewis-Säuren und Phosphorpentoxid, unter Bildung eines 1-(2-geschützten-Amino-4-thiazolyl)-propen-1,3-dicarbonsäuremonoesters (VI) entsprechend dem Reaktionsschema:

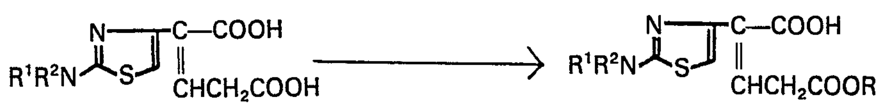

(V) (VI)

worin R eine esterbildende Gruppe, ausgewählt under (C1—C5)-Alkyl, (C2—C8)-Alkenyl, (C1—C5)-Alkyl, substituiert durch Halogen oder (C1—C5)-Alkoxy oder Sulfonyl, Benzyl, Benzyl, substituiert durch (C1—C5)-Alkyl oder (C1—C5)-Alkoxy oder Nitro oder Phenyl, bedeutet, $R^1$ eine Amino-Schutzgruppe, ausgewählt

unter (C1—C5)-Alkanoyl, Halo-(C1—C5)-alkanoyl, (C1—C5)-Alkoxycarbonyl, (C8—C15)-Aralkoxycarbonyl und (C13—C20)-Polyarylmethyl oder Tri-(C1—C5)-alkylsilyl, bedeutet und $R^2$ Wasserstoff bedeutet.

3. Verfahren nach Anspruch 2, dadurch gekennzeichnet, daß die Reaktion in einem Lösungsmittel, ausgewählt unter Kohlenwasserstoffen, Halogen-Kohlenwasserstoffen, dem Reagensalkohol und ihren Gemischen, durchgeführt wird.

4. Verfahren nach Anspruch 2, dadurch gekennziechnet, daß die Reaktion bei −10°C bis 80°C während einer Zeit von 0,3 Stunden bis 12 Tagen durchgeführt wird.

5. Verbindungen der Formeln VII und VIII

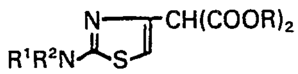

(VII)

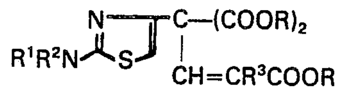

(VIII)

worin R, $R^2$ und $R^3$ die gleichen Bedeutungen wie in Anspruch 1 oder 2 besitzen und worin $R^1$ Wasserstoff oder eine Amino-Schutzgruppe wie in Anspruch 1 oder 2 angegeben bedeutet.

6. Verbindung nach Anspurch 5, dadurch gekennzeichnet, daß R (C1—C5)-Alkyl, (C1—C5)-substituiertes Alkyl, (C2—C8)-Alkenyl, Benzyl oder substituiertes Benzyl bedeutet.

7. Verbindung nach Anspruch 5, dadurch gekennzeichnet, daß $R^1$ und $R^2$ je Wasserstoff, (C1—C5)-Alkanoyl, (C1—C5)-Haloalkanoyl, (C1—C5)-Alkoxycarbonyl, (C8—C15)-Aralkoxycarbonyl, (C13—C20)-Polyarylmethyl oder Tri-(C1—C5)-alkylsilyl bedeuten.

## Revendications

1. Procédé de fabrication de monoesters d'acides aminothiazolylpropènedicarboxyliques, caractérisé par les étapes consistant à:

1) faire réagir un diester d'acide haloacétylmalonique (I) avec la thiourée, pour donner un diester d'acide (amino-2 thiazolyl-4)-2 malonique (II), conformément au schéma réactionnel:

$HalCH_2COCH(COOR)_2$ 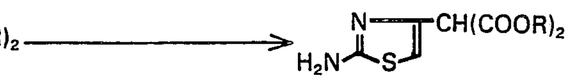

(I) (II)

où: Hal représente un atome d'halogène; et les R sont des groupes de formation d'esters, identiques ou différents, choisis parmi alkyle en C1—C5; alcényle en C2—C8, alkyle en C1—C5 substitué par halogène ou alcoxy en C1—C5 ou sulfonyle; benzyle; benzyle substitué par alkyle en C1—C5 ou alcoxy en C1—C5 ou nitro ou phényle;

2) faire réagir le composé (II) avec un réactif de protection du groupe amino, pour donner un diester d'acide (amino protégé-2 thiazolyl-4)-2 malonique (III), conformément au schéma réactionnel:

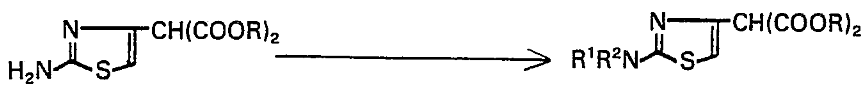

(II) (III)

où: R a la signification mentionée ci-dessus; $R^1$ représente un groupe protectueur d'amino choisi parmi alcanoyle en C1—C5; haloalcanoyle en C1—C5; (alcoxy en C1—C5)carbonyle; (aralcoxy en C8—C15)-carbonyle; (polyaryl en C13—C20)méthyle et tri(alkyle en C1—C5)silyle; et $R^2$ représente hydrogène;

3) faire réagir le composé (III) avec un ester d'acide haloalcénoïque dans un solvant aprotique et en présence d'une base forte. pour donner un tri- ou tétraester d'acide (amino protégé-2 thiazolyl-4)-1 propène-2 tricarboxylique-1,1,3 ou tétracarboxylique-1,1,3,3 (IV), conformément au schéma réactionnel:

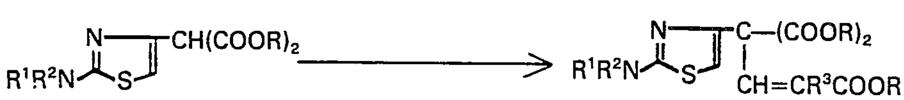

(III) (IV)

où: Hal, R, $R^1$ et $R^2$ ont les significations mentionnées ci-dessus; et $R^3$ représente hydrogène ou un groupe COOR;

4) soumettre le composé (IV) à une hydrolyse et une décarboxylation, pour donner une acide (amino protégé-2 thiazolyl-4)-1 propène-dicarboxylique-1,3 (V), conformément au schéma réactionnel:

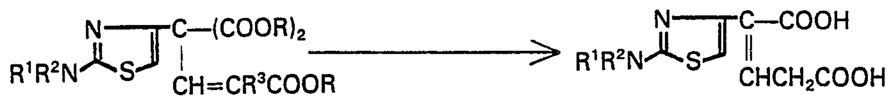

(IV) (V)

où: R, $R^1$ et $R^2$ ont les significations mentionnées ci-dessus;

5) faire réagir le composé (V) avec un alcool choisi parmi alcanol en C1—C5; alcénol en C2—C8; alcanol en C1—C5 substitué par halogène ou alcoxy en C1—C5 ou sulfonyle; alcool benzylique; ou alcool benzylique substitué par alkyle en C1—C5 ou alcoxy en C1—C5 ou nitro ou phényle; en présence d'un réactif de monoestérification choisi dans le groupe des acides minéraux, des acides alcanesulfoniques, des acides arylsulfoniques, des acides de Lewis, et du pentoxyde de phosphore, pour donner un monoester d'acide (amino protégé-2 thiazolyl-4)-1 propène-dicarboxylique-1,3 (VI), conformément au schéma réactionnel:

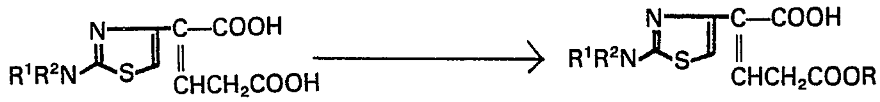

(V) (VI)

où: $R^1$ et $R^2$ ont les significations mentionnées ci-dessus; et R représente un groupe de formation d'ester choisi parmi alkyle en C1—C5; alcényle en C2—C8, alkyle en C1—C5 substitué par halogène ou alcoxy en C1—C5 ou sulfonyle; benzyle; ou benzyle substitué par alkyle en C1—C5 ou alcoxy en C1—C5 ou nitro ou phényle.

2. Procédé de fabrication de monoesters d'acides aminothiazolylpropènedicarboxyliques, caractérisé par l'étape consistant à: faire réagir un acide (amino protégé-2 thiazolyl)-1 propène-dicarboxylique-1,3 (V) avec un alcool choisi parmi alcanol en C1—C5; alcénol en C2—C8; alcanol en C1—C5 substitué par halogène ou alcoxy en C1—C5 ou sulfonyle; alcool benzylique; ou alcool benzylique substitué par alkyle en C1—C5 ou alcoxy en C1—C5 ou nitro ou phényle; en présence d'un réactif de monoestérification choisi dans le groupe des acides minéraux, des acides alcanesulfoniques, des acides arylsulfoniques, des acides de Lewis, et du pentoxyde de phosphore, pour donner un monoester d'acide (amino protégé-2 thiazolyl-4)-1 propène-dicarboxylique-1,3 (VI), conformément au schéma réactionnel:

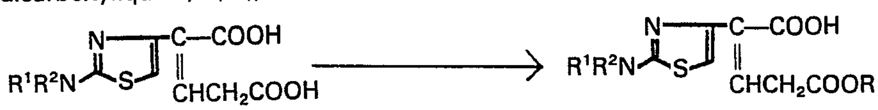

(V) (VI)

où: R représente un groupe de formation d'ester choisi parmi alkyle en C1—C5; alcényle en C2—C8, alkyle en C1—C5 substitué par halogène ou alcoxy en C1—C5 ou sulfonyle; benzyle; benzyle substitué par alkyle en C1—C5 ou alcoxy en C1—C5 ou nitro ou phényle; $R^1$ représente un groupe protecteur d'amino choisi parmi alcanoyle en C1—C5; haloalconyle en C1—C5; (alcoxy en C1—C5)carbonyle; (aralcoxy en C8—C15)-carbonyle; (polyaryl en C13—C20)méthyle ou tri(alkyle en C1—C5)silyle; et $R^2$ représente hydrogène.

3. Procédé selon la revendication 2, suivant lequel on effectue la réaction dans un solvant choisi parmi les hydrocarbures, les hydrocarbures halogénés, l'alcool réactif, et leurs mélanges.

4. Procédé selon la revendication 2, suivant lequel on effectue la réaction à une température allant de −10° à 80°C, pendant une période allant de 0,3 heure à 12 jours.

5. Composés représentés par les formules (VII) et (VIII):

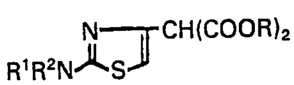

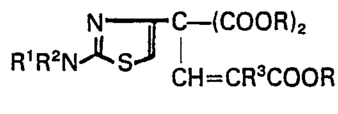

(VII) (VIII)

où: R, $R^2$ et $R^3$ ont les mêmes significations qu'à la revendication 1 ou 2; et $R^1$ peut représenter hydrogène ou un groupe protecteur d'amino tel que décrit à la revendication 1 ou 2.

6. Composé selon la revendication 5, dans lequel R représente alkyle en C1 à C5, alkyle en C1 à C5 substitué, alcényle en C2 à C8, benzyle, ou benzyle substitué.

7. Composé selon la revendication 5, dans lequel $R^1$ et $R^2$ représentent chacun, hydrogène, alcanoyle en C1—C5; haloalcanoyle en C1—C5; (alcoxy en C1—C5)carbonyle; (aralcoxy en C8—C15)carbonyle; (polyaryl en C13—C20)méthyle ou tri(alkyle en C1—C5)silyle.